**Europäisches Patentamt**

**European Patent Office**

**Office européen des brevets**

(19)

(11) Veröffentlichungsnummer : **0 078 430 B2**

(12)
# NEUE EUROPÄISCHE PATENTSCHRIFT

(45) Veröffentlichungstag der neuen Patentschrift :
**10.02.93 Patentblatt 93/06**

(51) Int. Cl.$^5$ : **A61K 9/18,** A61K 31/44, A61K 47/00

(21) Anmeldenummer : **82109572.6**

(22) Anmeldetag : **16.10.82**

(54) **Verfahren zur Herstellung von festen, schnellfreisetzenden Arzneizubereitungen mit Dihydropyridinen.**

Die Akte enthält technische Angaben, die nach dem Eingang der Anmeldung eingereicht wurden und die nicht in dieser Patentschrift enthalten sind.

(30) Priorität : **29.10.81 DE 3142853**
**16.02.82 DE 3205399**

(43) Veröffentlichungstag der Anmeldung :
**11.05.83 Patentblatt 83/19**

(45) Bekanntmachung des Hinweises auf die Patenterteilung :
**23.04.86 Patentblatt 86/17**

(45) Bekanntmachung des Hinweises auf die Entscheidung über den Einspruch :
**10.02.93 Patentblatt 93/06**

(84) Benannte Vertragsstaaten :
**AT BE CH DE FR GB IT LI LU NL SE**

(56) Entgegenhaltungen :
**EP-A- 0 001 247**
**EP-A- 0 004 650**
**DE-A- 2 200 778**
**DE-A- 2 822 822**
**DE-A- 2 950 154**
**FR-A- 2 121 805**
**JP-A-80 142 209**
**PHARM.IND., vol. 38, no. 12, 1976; pp. 1181-1185**
**PHARM.IND., vol. 48, no. 11a, 1978; pp. 1255-1263**
**CHEM.PHARM.BULLETIN, vol. 29, no. 6, 1980; SUGIMOTO et al., pp. 1715-1726**
**DRUG.DEV. & IND.PHARM., vol. 6, no. 2, 1980; pp. 137-160**

(73) Patentinhaber : **BAYER AG**
**W-5090 Leverkusen 1 Bayerwerk (DE)**

(72) Erfinder : **Hegasy, Ahmed, Dr.**
**Tempelhofer Str. 57**
**W-5090 Leverkusen 1 (DE)**

**EP 0 078 430 B2**

**Beschreibung**

Die vorliegende Erfindung betrifft ein Verfahren zur Herstellung von schnell resorbierbaren festen Arzneizubereitungen, die Dihydropyridine und Polyvinylpyrrolidon enthalten.

Nifedipin (1,4-Dihydro-2,6-dimethyl-4-(o-nitrophenyl)-pyridin-3,5-dicarbonsäure-dimethylester) ist ein bekannter kreislaufbeeinflussender Wirkstoff aus der Stoffklasse der Dihydropyridine (vgl. britisches Patent 1 173 862). Aufgrund seiner extremen Lichtempfindlichkeit sowie seiner extrem niedrigen Löslichkeit in wässrigen Medien treten bei der galenischen Zubereitung von Arzneispezialitäten eine Reihe von Schwierigkeiten auf, wie aus zahlreichen Patentanmeldungen und Patenten für spezielle Formulierungen dieses Wirkstoffes ersichtlich ist.

Das US-Patent 3 784 684 betrifft z.B. spezielle Gelatinebeisskapseln, die Nifedipin in gelöster Form enthalten, um die Coronarwirkung von Nifedipin vorteilhaft zu nutzen.

In dem Britischen Patent 1 456 618 werden feste Arzneizubereitungen beschrieben und beansprucht, die eine gute Bioverfügbarkeit des Nifedipins gewährleisten soll.

In der DT-A 2 822 882 werden ebenfalls feste Arzneizubereitungen beschrieben, bei denen durch den Einsatz bestimmter Lösungsmittel und oberflächenaktiver Substanzen die Schwerlöslichkeit von Nifedipin kompensiert werden soll.

Auch in der EP-A 1 247 soll die Resorbierbarkeit von Nifedipin durch die Verwendung von Polyethylenglykol (PEG) und bestimmter poröser Trägersubstanzen verbessert werden. In dieser Anmeldung wird auch beschrieben, dass die schlechte Löslichkeit von Nifedipin ausgeglichen werden kann, durch die Bildung von Copräzipitaten aus Nifedipin und Polyvinylpyrrolidon (PVP), in der das Nifedipin in amorpher Form vorliegt.

Diese Copräzipitate werden hergestellt durch Lösen von Nifedipin und PVP in organischen Lösungsmitteln mit. anschliessender Verdampfung des Lösungsmittels um eine Blasige Masse zu erhalten (vgl. DT-A 2 822 882). Eine solche Verdampfung des organischen Lösungsmittels lässt sich technisch nur mit grossem Aufwand durchführen, da die PVP-Masse die organischen Lösungsmittel stark bindet und kurz vor dem Trocknen sehr viskos wird. Es entsteht eine voluminöse schaumige Masse, die kurz vor Ende der Trocknung sehr zäh ist, nicht mehr gerührt und nur schwierig weiterverarbeitet werden kann. Ein weiterer Nachteil bei der Verwendung von üblichen PVP-Copräzipitaten bei der Tablettenherstellung ist die Tatsache, dass dieses Copräzipitat sich mit anderen Hilfsstoffen nur mischen lässt, aber nicht mehr ohne weiteres mit wässrigen Lösungen granuliert werden kann. Eine solche einfache Mischung zwischen dem PVP-Copräzipitat und anderen Hilfsstoffen neigt jedoch zu Entmischungen bei der weiteren maschinellen Verarbeitung, z.B. zu Tabletten oder beim Abfüllen in Kapseln. Dies kann letzlich zu Arzneizubereitungen mit sehr unterschiedlichem Wirkstoffgehalt in den einzelnen Tabletten oder Kapseln führen (mangelnde « Content Uniformity »), was bei einer hochwirksamen Substanz wie Nifedipin äusserst unerwünscht ist. Darüber hinaus sind die zur Auswahl stehenden zusätzlichen Hilfsstoffe sehr begrenzt, insbesondere für die Tablettenherstellung weil PVP gleichzeitig als Bindemittel wirkt und durch die Anwesenheit von grösseren Mengen von PVP (30-100 mg pro Tablette oder Kapsel) der Zerfall der Tabletten bzw. Kapseln verhindert wird.

Nimodipin (1,4-Dihydro-2,6-dimethyl-4-(3'-nitrophenyl)-pyridin-3-β-methoxyethylester-5-isopropylester) ist ebenfalls ein bekanntes cerebral wirkendes Dihydropyridin (vergl. D-A 2 815 578). Auch diese Verbindung lässt sich aufgrund ihrer physikalisch-chemischen Eigenschaften nur schwierig zu galenischen Zubereitungsformen verarbeiten. Nimodipin ist z.B. in wässrigen Medien noch wesentlich schlechter löslich als Nifedipin.

Die vorliegende Erfindung betrifft ein Verfahren zur Herstellung von neuen festen schnell freisetzenden Zubereitungen mit Dihydropyridinen, die diese Nachteile bisher bekannter Zubereitungen nicht mehr aufweisen. Sie betrifft insbesondere die Herstellung von schnell resorbierbaren festen Arzneizubereitungen mit gleichmässigem Wirkstoffgehalt, dessen relative Standardabweichung höchstens 3%, insbesondere höchstens 2,5 beträgt, enthaltend 1 Gew.-Teil Dihydropyridin, 2.0-6,0 Gew.-Teile Polyvinylpyrrolidon (PVP) mit einem mittleren Molekulargewicht von 15000-50000 und absorptionsfähige Trägerstoffe aus der Gruppe, 3,5-15 Gew.-Teile Cellulose, 0,25-4,0 Gew.-Teile Stärke und 0,25-4,0 Gew.-Teile quervernetztes unlösliches Polyvinylpolypyrrolidone (PVPP), sowie gegebenenfalls weitere übliche Hilfs- und Trägerstoffe.

Die Herstellung dieser festen Arzneizubereitungen erfolgt, indem man 1 Gewichtsteil der Dihydropyridine Nifedipin oder Nimodipin und 2 - 6 Gewichtsteile PVP in 2 - 20 Gewichtsteile Ethanol, Aceton, Methylenchlorid oder Chloroform oder Gemischen dieser Lösungsmittel, in denen sich die beiden festen Bestandteile gerade noch lösen lassen, auflöst und diese Lösung mit 4 - 23 Gewichtsteilen, insbesondere mit 5,25 - 17 Gewichtsteilen fester Trägerstoffe mit großer Absorptionsfähigkeit bestehend aus einem Gemisch von Cellulose, Stärke und quervernetztem unlöslichem Polyvinylpyrrolidon (PVPP) mit großer Absorptionsfähigkeit granuliert, und dieses Granulat gegebenenfalls anschließend mit üblichen Hilfs- und Trägerstoffen zu festen Arzneizubereitungen weiterverarbeitet.

Vorzugsweise werden die Lösungsmittel in einer Menge von 8 - 16 Gewichtsteilen bezogen auf das Dihydropyridin eingesetzt.

Als fester Trägerstoff mit grosser Absorptionsfähigkeit eignet sich besonders eine Pulvermischung aus 4-12 Gew.-Teilen Cellulose, 0,25-2,0 Gew.-Teilen Stärke und 1-3 Gew.-Teilen quervernetztes unlösliches PVPP.

Als übliche Hilfs- und Trägerstoffe, die zur Weiterverarbeitung zu festen Arzneizubereitungen verwendet werden können, seien vorzugsweise genannt: Milchzucker, Puderzucker, Mannit, Glykokoll und Calciumcarbonat.

Als feste Arzneizubereitungen seien vorzugsweise genannt: Tabletten, Pillen, Dragees, Granulate, Pulver, Kapseln und Sachets.

Es war nicht vorhersehbar, dass die so erhaltenen Arzneizubereitungen eine sehr gute Bioverfügbarkeit und gleichzeitig eine gute Content-Uniformity besitzen. Die durch das erfindungsgemässe Verfahren erhaltenen Granulate, bestehend aus der mit Wirkstoff-PVP-Lösung benetzten Pulvermischung, lassen sich problemlos trocknen, sieben, weiterverarbeiten, mit anderen Hilfs- und Trägerstoffen vermischen und zu Tabletten pressen.

Im Hinblick auf die aus dem Stand der Technik bekannten zahlreichen Versuche Beeignete Arzneizubereitungen mit Dihydropyridin herzustellen, war es nicht vorhersehbar, dass durch das erfindungsgemässe Verfahren, welches leicht und ohne technischen Aufwand durchgeführt werden kann, neue und wertvolle Arzneizubereitungen mit diesen Wirkstoffen erhalten werden, die sich durch eine gute Bioverfügbarkeit auszeichnen, und deren Wirkstoffgehalt nur eine maximale Standardabweichung von 3%, insbesondere von 2,5% beträgt (vgl. das Lehrbuch:

Pharmazeutische Technologie, herausgegeben von Sucker, Fuchs und Speises, Seite 32 bis 37 sowie US Pharmacopoea XX, Seite 955 bis 957).

Zum Nachweis der vorteilhaften Eigenschaften der erfindungsgemäss hergestellten besten Zubereitungen wurden gemäss den Beispielen A bis F Tabletten nach herkömmlichen Methoden hergestellt, in dem zunächst ein festes Copräzipitat aus Nifedipin und PVP durch Abdampfen des Lösungsmittels hergestellt wurde und dieses feste Granulat dann mit üblichen Hilfs- und Trägerstoffen gemischt bzw. granuliert und zu festen Tabletten weiterverarbeitet wurde. In den erfindungsgemässen Beispielen 1 bis 8 wurde dagegen das Copräzipitat aus Nifedipin und PVP bzw. aus Nimodipin und PVP nicht isoliert, sondern als Lösung mit einer Mischung fester Trägerstoffe granuliert und dieses Granulat anschliessend in üblicher Weise zu Tabletten gepresst.

Die folgende Tabelle zeigt, dass die erfindungsgemässen festen Formulierungen sehr schnell und vollständig resorbiert werden (gute Freisetzung) und gleichzeitig nur eine sehr geringe relative Standardabweichung des Wirkstoffgehalts zeigen (gute Content Uniformity).

### Tabelle

| Beispiel Nr. | Beispiele nach bekannten Methoden | | | | | | Erfindungsgemässe Beispiele | | | |
|---|---|---|---|---|---|---|---|---|---|---|
| | A | B | C | D | E | F | 1 | 2 | 3 | 4 |
| Standardabweichung der Tablettengewichte | 1,7% | 1,0% | 1,1% | 1,7% | – | – | 1,0% | 0,7% | 0,7% | 0,8% |
| Abweichung des Nifedipingehaltes in mg in Einzeltabletten (10 Tabletten) | 8,86–10,12 | 8,04–10,32 | 8,75–10,15 | 8,44–9,65 | 10,01–10,83 | 9,55–10,03 | 10,03–10,31 | 10,30–10,55 | 10,49–10,81 | 9,98–10,53 |
| Standardabweichung des Nifedipingehaltes pro Tablette | 4,1% | 6,7% | 5,1% | 4,7% | 2,4% | 1,8% | 1,0% | 0,774% | 1,088% | 1,413% |
| Nifedipin-Freisetzung* 50% freigesetzt nach Minuten | ca. 10′ | ca. 10′ | ca. 10′ | ca. 10′ | <10′ | 30′–40′ | <5′ | <10′ | <10′ | <10′ |

* Die Freisetzungsprüfung erfolgt nach der USP-Padelle-Methode.

Beispiele für die Herstellung von festen Nifedipinzubereitungen nach bekannten Methoden

Die Ansätze der Beispiele A bis F sowie Beispiel 1 sind auf die Herstellung von jeweils 10 000 Tabletten bezogen. Die Beispiele 2 bis 4 sind jeweils 15 000 Tabletten berechnet.

Beispiel A

100 g Nifedipin und 400 g PVP 25 (mittleres Molgewicht 25 000) werden in 1500 g Methylenchlorid gelöst. Beim Verdampfen des Lösungsmittels entsteht zunächst eine sehr viskose nicht rührfähige Masse, die nach einiger Zeit in eine schaumige glasige Masse übergeht. Dieses Copräzipitat wird über ein Oszillationssieb auf eine maximale Korngrösse von 1,0 mm gebracht. In einem separaten Arbeitsgang wird aus 1050 g Avicel (Cellulose), 350 g Maisstärke und 50 g mit Wasser verkleisteter Maisstärke ein Granulat hergestellt. Dieses Granulat wird mit 500 g des Nifedipincopräzipitates, mit 4 g Magnesiumstearat und 246 g unlöslichem quervernetztem PVPP gemischt. Aus dieser Mischung werden Tabletten mit einem Gewicht von 220 mg hergestellt, die einen mittleren Nifedipingehalt von 10 mg haben.

Beispiele B und C

Die in gleicher Weise wie unter A hergestellten Copräzipitate werden über ein Oszillationssieb auf eine maximale Korngrösse von 0,8 mm bei Beispiel B bzw. 0,6 mm bei Beispiel C gebracht und anschliessend in gleicher Weise wie Beispiel A zu Tabletten weiterverarbeitet.

Beispiel D

Das analog Beispiel A hergestellte Copräzipitat wird mittels einer Hammermühle zerkleinert und anschliessend wie im Beispiel A zu Tabletten weiterverarbeitet.

Beispiel E

100 g Nifedipin und 400 g PVP 25 werden in 1200 g Methylenchlorid gelöst und die Lösung unter Vakuum getrocknet. Das erhaltene Copräzipitat wird direkt mit einem Granulat, welches aus 100 g Cellulose, 450 g Stärke und 50 g mit Wasser verkleisterter Stärke hergestellt wurde, und mit 5 g Magnesiumstearat, 195 g Stärke und 200 g unlöslichem Polyvinylpolypyrrolidon vermischt und dieses Gemisch dann zu 240 mg schweren Tabletten gepresst.

Beispiel F

Ein analog Beispiel E hergestelltes Copräzipitat wurde unmittelbar mit dem Gemisch aus Cellulose und Stärke gemischt und mit der verkleisterten Stärke, d.h. wässrig granuliert. Und anschliessend analog Beispiel E zu Tabletten gepresst. Die so hergestellten Tabletten besitzen zwar eine verbesserte Content Uniformity, ergeben aber unbefriedigende Freisetzungswerte.

Ausführungsbeispiele nach dem erfindungsgemässen Verfahren

Beispiel 1

100 g Nifedipin und 400 g PVP 25 werden in 1800 Methylenchlorid gelöst. In einem Granuliergerät werden 1050 g Cellulose, 200 g Stärke und 100 g unlösliches PVPP trocken gemischt und mit der Nifedipin-PVP-Lösung granuliert. Das erhaltene feuchte Granulat wird getrocknet und gesiebt, dann werden 146 g unlösliches PVPP, 200 g Stärke und 4 g Magnesiumstearat zugegeben, gemischt und diese Mischung zu Tabletten von 220 mg gepresst. Diese Tabletten zeichnen sich durch eine sehr gute Content Uniformity und vorteilhafte Freisetzung aus.

Beispiel 2

Analog Beispiel 1 wurden 150 g Nifedipin und 600 g PVP 25 in 2200 g Methylenchlorid gelöst. Die Lösung wurde zur Granulierung einer Mischung von 1575 g mikrokristalliner Cellulose 600 g Maisstärke und 300 g unlösliches PVPP granuliert. Nach Trocknung und Sieben des Granulates wird dieses mit 6 g Magnesiumstearat und 69 g unlösliches PVPP gemischt und zu Tabletten von 220 mg Gewicht gepresst.

Beispiel 3

150 g Nifedipin und 600 g PVP 25 wurden in 2100 g Methylenchlorid gelöst. Diese Lösung wurde zur Gra-

4

nulierung einer Mischung aus 1575 g mikrokristalliner Cellulose, 600 g Maisstärke und 150 g unlösliches PVPP benutzt. Nach Trocknung und Sieben des Granulates wird dieses mit 6 g Magnesiumstearat und 219 g unlöslichen PVPP gemischt und zu Tabletten von 220 mg Gewicht gepresst.

Beispiel 4

Folgende Mengen sind für die Herstellung von 100 000 Tabletten mit je 10 mg Nifedipin berechnet: 1 kg Nifedipin und 4 kg PVP 25 wurden in 7 kg Aceton gelöst. Die Lösung wurde zur Granulation einer Mischung von 10,5 kg mikrokristalliner Cellulose (Avicel®), 2 kg Stärke und 1 kg unlöslichem PVPP benutzt. Die Masse wurde unter Vakuum getrocknet, gesiebt und mit 1,46 kg unlöslichem PVPP, 2 kg Stärke und 0,04 kg Magnesiumstearat gemischt. Die Mischung wurde zu Tabletten von 220 mg Gewicht und einem Durchmesser von 9 mm gepresst. 20 kg Tabletten wurden besprüht mit einer Suspension aus 0,3 kg Hydroxypropylmethylcellulose, 0,1 kg Polyethylenglykol 4000, 0,09 kg Titandioxid, 0,01 kg rotes Eisenoxid in 6,17 kg Wasser.

Beispiel 5

Folgende Mengen sind für die Herstellung von 250 000 Tabletten mit je 20 mg Nifedipin berechnet: 5 kg Nifedipin und 20 kg PVP 25 wurden gelöst in einer Mischung aus 35 kg Aceton und 10 kg Methylenchlorid. Die Lösung wurde zur Granulation einer Mischung aus 52,5 kg mikrokristalliner Cellulose, 10 kg Stärke und 5 kg unlöslichem PVPP benutzt. Nach Trocknung unter Vakuum und Siebung wurden 7,3 kg unlösliches PVPP, 10 kg Stärke und 0,2 kg Magnesiumstearat zugemischt. Die Mischung wurde zu Tabletten von 440 mg Gewicht gepresst. 100 kg Tabletten wurden lackiert durch Besprühen mit einer Suspension aus 1,5 kg Hydroxypropyl-methylcellulose, 0,5 kg PEG 4000, 0,4 kg Titandioxid, 0,1 kg rotes Eisenoxid und 30,83 kg Wasser.

Beispiel 6

Folgende Mengen sind für die Herstellung von 20000 Tabletten mit je 30 mg Nimodipin berechnet: 0,6 kg Nimodipin und 1,5 kg PVP 25 wurden in 1,4 kg Aceton gelöst. Die Lösung wurde zur Granulation einer Mischung von 2,85 kg mikrokristalline Cellulose, 0,150 kg Stärke und 0,6 kg unlöslichem PVPP benutzt. Die Masse wurde unter Vakuum getrocknet, gesiebt und mit 0,288 kg unlöslichem PVPP, 0,566 kg Stärke und 0,011 kg Magnesiumstearat gemischt. Die Mischung wurde zu Tabletten von 330 mg Gewicht und einem Durchmesser von 10 mm gepresst. 6 kg Tabletten wurden lackiert durch Besprühen mit einer Suspension aus 0,225 kg Hydroxypropylmethylcellulose, 0,075 kg Polyethylenglykol 4000 und 0,075 kg Titandioxid in 4,625 kg Wasser. Die Einzeltablettenanalysen des Gehalts an Nimodipin lagen zwischen 29,78 mg und 31,18 mg mit einer relativen Standardabweichung von 1,156%.

Beispiel 7

Ein Ansatz von 26,4 kg Tablettenmischung, ausreichend für 60 00 Tabletten mit je 40 mg Nimodipin wurde folgendermassen hergestellt:
2,4 kg Nimodipin und 6,0 kg PVP 25 wurden in 5,76 kg Aceton solange gerührt bis eine klare viskose Lösung vorlag. In einem Granuliergerät wurden 11,4 kg mikrokristalline Cellulose, 0,6 kg Stärke und 2,4 kg unlösliches PVPP gemischt. Anschliessend wurde die oben angegebene Nimodipin enthaltende Lösung zugegeben. Nach Granulierung dieser Lösung mit den Pulverbestandteilen wurde die Masse im Vakuum getrocknet und anschliessend durch Sieben auf eine mittlere Korngrösse von 1,0 mm Durchmesser gebracht. Hierzu wurden 2,4 kg Stärke, 1,152 kg unlösliches PVPP und 0,48 kg Magnesiumstearat gemischt. Aus der Mischung wurden Tabletten mit einem Gewicht von 440 mg gepresst. 24 kg dieser Tabletten wurden durch Besprühen mit einer Suspension aus 0,54 kg Hydroxypropyl-methylcellulose, 0,18 kg Polyethylenglykol 4000 und 0, 18 kg Titandioxid in 11,1 kg Wasser lackiert. Der mittlere Gehalt an Nimodipin in den Einzeltabletten betrug 40,5 mg. Die relative Standardabweichung lag bei 2,21%.

**Patentansprüche**

1. Verfahren zur Herstellung von festen schnell freisetzenden Arzneizubereitungen enthaltend 1 Gewichtsteil der Dihydropyridine Nifedipin oder Nimodipin, 2 - 6 Gewichtsteile Polyvinylpyrrolidon mit einem mittleren Molekulargewicht von 15.000 - 50.000 und absorptionsfähige Trägerstoffe aus der Gruppe 3,5 - 15 Gewichtsteile Cellulose, 0,25 - 4,0 Gewichtsteile Stärke und 0,25 - 4,0 Gewichtsteile quervernetztes unlösliches Polyvinylpyrrolidon, sowie gegebenenfalls weitere üblich Hilfs- und Trägerstoffe, dadurch gekennzeichnet, daß man den Dihydropyridinwirkstoff und das Polyvinylpyrrolidon in 2 - 20 Gewichtsteilen Ethanol, Aceton, Methylenchlorid oder Chloroform oder Gemischen dieser Lösungsmittel, in denen sich die beiden Bestandteile gerade noch lösen lassen, auflöst und diese Lösung mit 4 - 23 Gewichtsteilen der festen absorptionsfähigen Trägerstoffe, bestehend aus einem Gemisch von Cellulose, Stärke und quervernetztem unlöslichem Polyvinylpolypyrrolidon granuliert und dieses Granulat gegebenenfalls anschließend mit üblichen Hilfs- und Trägerstoffen zu festen Arzneizubereitungen weiter verarbeitet.

2. Verfahren gemäß Anspruch 1, dadurch gekennzeichnet, daß man 5,25 - 17 Gewichtsteile fester absorptionsfähiger Trägerstoffe einsetzt.

3. Verfahren gemäß Anspruch 1, dadurch gekennzeichnet, daß man übliche Hilfs- und Trägerstoffe aus der Gruppe Milchzucker, Puderzucker, Mannit, Glycokoll und/oder Calciumcarbonat verwendet.

## Claims

1. Process for the production of solid rapid-release medicament preparations containing 1 part by weight of the dihydropyridines nifedipine or nimodipine, 2-6 parts by weight of polyvinylpyrrolidone having an average molecular weight of 15,000-50,000 and absorptive excipients from the group comprising 3.5-15 parts by weight of cellulose, 0.25-4.0 parts by weight of starch and 0.25-4.0 parts by weight of cross-linked insoluble polyvinylpyrrolidone, and if appropriate other customary auxiliaries and excipients, characterised in that the dihydropyridine active compound and the polyvinylpyrrolidone are dissolved in 2-20 parts by weight of ethanol, acetone, methylene chloride or chloroform or mixtures of these solvents in which the two constituents are just still able to dissolve and this solution is granulated with 4-23 parts by weight of the solid absorptive excipients, composed of a mixture of cellulose, starch and cross-linked insoluble polyvinylpolypyrrolidone, and these granules are optionally subsequently further processed with customary auxiliaries and excipients to give solid medicament preparations.

2. Process according to Claim 1, characterised in that 5.25-17 parts by weight of solid absorptive excipients are employed.

3. Process according to Claim 1, characterised in that customary auxiliaries and excipients from the group comprising lactose, powdered sugar, mannitol, glycine and/or calcium carbonate are used.

## Revendications

1. Procédé de préparation de compositions thérapeutiques solides à libération rapide contenant 1 partie en poids des dihydropyridines nifédipine ou nimodipine, 2 à 6 parties en poids de polyvinylpyrrolidone de poids moléculaire moyen 15 000 à 50 000 et des véhicules absorbants du groupe formé par 3,5 à 15 parties en poids de cellulose, 0,25 à 4,0 parties en poids d'amidon et 0,25 à 4,0 parties en poids de polyvinylpolypyrrolidone réticulée insoluble, et éventuellement d'autres produits auxiliaires et véhicules usuels, caractérisé en ce que l'on dissout la dihydropyridine active et la polyvinylpyrrolidone dans 2 à 20 parties en poids d'éthanol, d'acétone, de chlorure de méthylène ou de chloroforme ou d'un mélange de ces solvants, dans lequel les deux constituants sont encore tout juste solubles, et on utilise cette solution pour la mise en granulés de 4 à 23 parties en poids des véhicules solides absorbants consistant en un mélange de cellulose, d'amidon et de polyvinylpolypyrrolidone réticulée insoluble, après quoi, le cas échéant, on met ces granulés sous la forme de compositions thérapeutiques solides à l'aide de produits auxiliaires et véhicules usuels.

2. Procédé selon la revendication 1, caractérisé en ce que l'on utilise de 5,25 à 17 parties en poids de véhicules absorbants solides.

3. Procédé selon la revendication 1, caractérisé en ce que l'on utilise des produits auxiliaires et véhicules usuels du groupe du lactose, du sucre en poudre, du mannitol, du glycocolle et/ou du carbonate de calcium.